# EUROPÄISCHE PATENTSCHRIFT

(11) **EP 1 005 648 B1**
(45) Veröffentlichungstag und Bekanntmachung des Hinweises auf die Patenterteilung: **08.12.2004**
(21) Anmeldenummer: 98946394.8
(22) Anmeldetag: 20.08.1998
(51) Int. Cl.: G01N 33/22, G01N 27/22

(54) **VERFAHREN ZUR VERBRENNUNGSLOSEN MESSUNG DES BRENNWERTES VON BRENNGAS**
METHOD FOR MEASURING WITHOUT COMBUSTION THE HEAT VALUE OF FUEL GAS
PROCEDE PERMETTANT DE MESURER SANS COMBUSTION LE POUVOIR CALORIFIQUE D'UN GAZ COMBUSTIBLE

(30) Priorität: 22.08.1997 DE 19736528
(43) Veröffentlichungstag der Anmeldung: 07.06.2000
(73) Patentinhaber: E.ON Ruhrgas AG, 45138 Essen (DE); N.V. NEDERLANDSE GASUNIE, 9700 MA Groningen (NL)
(72) Erfinder: SCHLEY, Peter, D-45131 Essen (DE); JAESCHKE, Manfred, D-46286 Dorsten (DE); KLEINRAHM, Reiner, D-44801 Bochum (DE); JANSSEN-VAN ROSMALEN, Renee, NL-9301 WB Roden (NL); SCHOUTEN, Jan, A., NL-1141 GT Monnickendam (NL)
(74) Vertreter: Harlacher, Mechthild
(86) Internationale Anmeldenummer: PCT/EP1998/005304
(87) Internationale Veröffentlichungsnummer: WO 1999/010740

(56) Entgegenhaltungen:
- DE-A- 4 336 174
- US-A- 4 845 976
- US-A- 5 182 523
- Deutsche Norm DIN1343 (Januar 1990)

## Beschreibung

Die Erfindung bezieht sich auf ein Verfahren zur verbrennungslosen Messung des Brennwertes von Brenngas, das durch eine Brenngasleitung strömt und auf eine Verwendung des Verfahrens zur verbrennungslosen Messung und/oder Regelung der Wärmemengenzufuhr zur Gasverbrauchseinrichtungen, insbesondere zu Erdgasverbrauchseinrichtungen.

Die verbrennungslosen Verfahren zur Messung des Brennwertes bzw. zur Wärmemengenmessung haben gegenüber kalorimetrischen Verfahren, in denen eine kontrollierte Teilstrom-Verbrennung des übertragenen Gasstroms durchgeführt wird, den Vorteil, dass sie wesentlich kostengünstiger sind. Allerdings ist die technische Realisierung häufig aufwendig, abgesehen davon, dass Schwierigkeiten bei der Eichung auftreten können.

Zu den verbrennungslosen Messverfahren gehören indirekte und korrelative Verfahren. Bei den indirekten Verfahren wird die Gaszusammensetzung analysiert. Aus der Zusammensetzung des Gases kann dann mit den Brennwerten für die reinen Stoffe der Brennwert des Brenngases bestimmt werden. Diese Verfahren (z.B. die Gaschromatographie) liefern sehr genaue Ergebnisse, sind aber technisch kompliziert und daher für den Einsatz in beispielsweise Haushalten kaum geeignet. Zudem sind sie störanfällig.

Bei den korrelativen Verfahren zur Messung des Brennwertes bzw. zur Wärmemengenmessung wird ein Zusammenhang zwischen einer leicht messbaren physikalischen oder chemischen Messgröße und dem Brennwert ausgenutzt. Die technische Durchführung ist hierbei einfacher, jedoch werden die Reproduzierbarkeit und die Genauigkeit der Messung des Brennwertes bzw. der Wärmemenge in unerwünschtem Maße eingeschränkt.

Aus der DE-A-4 336174 ist ein korrelatives Verfahren zur verbrennungslosen Messung und Regelung der Wärmemengenzufuhr zu Gasverbrauchseinrichtungen beschrieben, bei dem der Volumenstrom, der Druck, die Temperatur, die Schaltgeschwindigkeit und die Dichte des Gases gemessen werden. Da relativ viele Messungen erforderlich sind, ist dieses Verfahren relativ aufwendig.

Aufgabe der Erfindung ist es, den Aufwand bei der korrelativen verbrennungslosen Messung des Brennwertes bzw. der Messung und/oder Regelung der Wärmemengenzufuhr zu Verbrauchseinrichtungen weiter zu verringern und insbesondere ein zuverlässiges und genaues Messverfahren zur Verfügung zu stellen.

Diese Aufgabe wird erfindungsgemäß gelöst durch ein Verfahren zur verbrennungslosen Messung des Brennwertes von Brenngas, das durch eine Brenngasleitung strömt, wobei
a) die Dielektrizitätskonstante des Brenngases unter Referenz- oder Betriebsbedingungen und die Dichte oder die Schallgeschwindigkeit des Brenngases unter Referenz- oder Betriebsbedingungen erfasst werden und
b) aus diesen zwei Parametern der Brennwert abgeleitet wird,
c) den Verfahrensschritten a) und b) mehrere Messzyklen vorgeschaltet werden, bei denen der Schritt a) mit mehreren Referenzgasen bekannten Brennwerts durchgeführt wird; aus dem Verhältnis der bei den Referenzgasen erfassten verschiedenen Messsignale eine der Zahl der Messzyklen entsprechende Zahl von Referenzsignalmustern in Zuordnung zu den bekannten Brennwerten gespeichert wird; und das Signalmuster aus einem späteren Messzyklus an Brenngas unbekannten Brennwertes mit den Referenzsignalmustem zur Zuordnung eines bestimmten Brennwertes verglichen wird.

Für die Messung der Dichte unter Referenz- bzw. Betriebsbedingungen existieren in der Praxis verschiedene bewährte Verfahren. Die Schallgeschwindigkeit bei Referenz- oder Betriebsbedingungen kann in einer separaten Messeinheit, beispielsweise über die Resonanzfrequenz von Wirbelpfeifen oder von Hohlkörpern oder eine Weg-Zeit-Messung z. B. in Ultraschallzählern erfasst werden. Die Dielektrizitätskonstante lässt sich auch bei Betriebsbedingungen kostengünstig und mit hoher Genauigkeit erfassen.

Folglich können die jeweils erforderlichen zwei Messungen ohne großen technischen Aufwand zuverlässig und genau durchgeführt werden, so dass die Verknüpfung der Messwerte entsprechende Ergebnisse für den Brennwert liefert. Der so ermittelte Brennwerk kann z. B. zur Steuerung von Verbrennungsprozessen verwendet werden.

Je nach Anwendungsfall kann der volumenbezogene Brennwert unter Referenz- oder Betriebsbedingungen, der spezifische (massenbezogene) Brennwert bzw. der molare Brennwert bestimmt werden.

insgesamt gibt es vier Variationen des erfindungsgemäßen Verfahrens zur Messung des Brennwertes von Brenngas. Bei der ersten Variante werden die Dichte und die Dielektrizitätskonstante unter Referenzbedingungen und bei der zweiten Variante die Schallgeschwindigkeit und die Dielektrizitätskonstante unter Referenzbedingungen gemessen. Diese beiden Varianten haben den Vorteil, dass die bei Referenzbedingungen arbeitenden Messgeräte besonders kostengünstig in der Anschaffung und einfach zu warten sind.

Bei der dritten Variante werden die Dichte und die Dielektrizitätskonstante unter Betriebsbedingungen und bei der vierten Variante die Schallgeschwindigkeit und die Dielektrizitätskonstante unter Betriebsbedingungen gemessen. Diese letzten beiden Varianten haben den Vorteil, dass keine Thermostate zur Herstellung von Referenzbedingungen benötigt werden. Schließlich sind bei der dritten und vierten Variante keine Druckreduzierungen zur Herstellung üblicher Referenzbedingungen erforderlich.

Dafür sind die Meßeinrichtungen zur Messung der Dielektrizitätskonstanten und der Dichte oder Schallgeschwindigkeit bei Betriebsbedingungen etwas kostspieliger in der Anschaffung als die entsprechenden Meßeinrichtungen für die ersten beiden Verfahrensvarianten. Außerdem ist die Auswertung der Meßgrößen zur Bestimmung des Brennwertes bei den letzten beiden Varianten des erfindungsgemäßen Verfahrens zur Messung des Brennwertes schwieriger als bei den ersten beiden varianten.

Zum Auffinden einer geeigneten Korrelation zwischen den gemessenen Parametern und dem Brennwert wird den jeweiligen Verfahrensschritten a) und b) wenigstens einmal mehrere Meßzyklen vorzuschalten, bei denen der Verfahrensschritt a) mit mehreren Referenzgasen bekannten Brennwerts durchgeführt wird. An dem Referenzgas werden dann die für die verschiedenen Varianten der Verfahren benötigten Meßgrößen erfaßt. In diesen Referenzzyklen wird aus dem Verhältnis der erfaßten verschiedenen Meßsignale eine der Zahl der Meßzyklen entsprechende Zahl von Referenzsignalmustem in Zuordnung zu den bekannten Brennwerten gespeichert. Das Signalmuster aus einem späteren Meßzyklus an Brenngas. unbekannten Brennwertes wird mit den Referenzsignalmustern zur Zuordnung eines bestimmten Brennwertes verglichen.

Zur Erhöhung der Referenzgenauigkeit sollte eine Vielzahl von Referenzzyklen durchgeführt werden, in denen nacheinander die verschiedenen Meßgrößen über den zu erwartenden Meßbereich variiert werden. Eine eindeutige und genaue Zuordnung eines bestimmten Brennwertes zu einem in einem Meßzyklus erfaßten Signalmuster eines Brenngases wird durch Interpolation der verschiedenen Referenzsignalmuster erzielt.

Ein wesentlicher Vorteil besteht darin, daß die Korrelation zwischen Brennwert und gemessenen Parametern mit Hilfe einer beliebigen Anzahl von Referenzzyklen für eine spezielle Anwendung nur einmal aufgefunden werden muß. Der ten hierbei möglichst getreu den später zu erwartenden Meßbedingungen gewählt werden. Es sollten also für alle Meßgrößen nur die tatsächlich in Frage kommenden Meßbereiche mit einer ausreichenden Genauigkeit als Referenzsignalmuster erfaßt werden.

Vorteilhafterweise werden die Dielektrizitätskonstante und die Dichte oder die Schallgeschwindigkeit unter Referenzbedingungen in einer gemeinsamen Meßumgebung erfaßt. Auf diese Weise wird nur eine Temperatur- und Druckmessung und folglich nur ein Thermostat zur Herstellung bzw. Einhaltung der Referenzbedingungen benötigt. Außerdem erhöhen einheitliche Referenzbedingungen für die Messung der Dielektrizitätskonstanten und der Dichte die Genauigkeit, mit der die Wärmemengenzufuhr bestimmt werden kann.

Ein bevorzugtes Ausführungsbeispiel ist dadurch gekennzeichnet, daß als Referenzbedingungen für die Messung der Dichte oder der Schallgeschwindigkeit und/oder der Dielektrizitätskonstanten Normbedingungen eingestellt werden.

Die Dielektrizitätskonstante kann bei einem Referenzdruck von wenigstens 1 Mpa besonders genau gemessen werden.

Die Meßgenauigkeit des Brennwertes kann dadurch weiter erhöht werden, daß im Schritt a) zusätzlich wenigstens eine der Meßgrößen Temperatur, Druck oder der Anteil mindestens eines Inertgases, vorzugsweise der Kohlenstoffdioxidanteil, erfaßt wird. Selbstverständlich kann die höchste Meßgenauigkeit bei der zusätzlichen Erfassung aller drei Meßgrößen erzielt werden.

Eine Weiterbildung der Erfindung ist dadurch gekennzeichnet, daß ein Teilstrom des Brenngases zur Messung der Dichte oder der Schallgeschwindigkeit unter Referenzbedingungen abgezweigt wird und daß an diesem Teilstrom, vorzugsweise nach der Dichte- oder Schallgeschwindigkeitsmessung, der Anteil mindestens eines Inertgases, vorzugsweise der' Kohlenstoffdioxidanteil, erfaßt wird.

Die erfindungsgemäße Aufgabe wird ferner gelöst durch die Verwendung des erfindungsgemäßen Verfahrens zur verbrennungslosen Messung und/oder Regelung der Wärmemengenzufuhr zu Gasverbrauchseinrichtungen, insbesondere zu Erdgasverbrauchseinrichtungen, wobei im Schritt a) zusätzlich das Brenngas bzw. ein Teilstrom des Brenngases durch einen Volumen- oder Massenzähler geleitet und das Volumen bzw. die Masse des zugeführten Brenngases gemessen wird.

Unter Gasverbrauchseinrichtungen werden alle privaten und industriellen Abnehmer sowie alle Übergabestellen oder dergleichen verstanden.

Erfindungsgemäß kann z. B. die Wärmemengenzufuhr zu Haushalten bereits aus drei Parametern, nämlich erstens dem Volumen oder der Masse, zweitens der Dielektrizitätskonstante und drittens der Dichte oder Schallgeschwindigkeit unter Referenz- oder Betriebsbedingungen abgeleitet werden. Der technische Aufwand und die Kosten hierfür sind minimal.

Zur Erhöhung der Genauigkeit können, wie bei der Messung des Brennwertes, beliebig viele weitere Meßgrößen erfaßt werden. Für Anwendungsfälle, in denen besonders hohe Meßgenauigkeiten erforderlich sind, z. B. für die Bestimmung der Wärmemengenzufuhr an Übergabestellen von Haupttransportleitungen mit hohem Gasdurchsatz, ist es vorteilhaft, zusätzlich zu den obigen drei Parametern den Druck, die Temperatur und den Kohlenstoffdioxidanteil zu erfassen.

Im folgenden wird die Verwendung des erfindungsgemäßen Verfahrens zur verbrennungslosen Messung und/oder Regelung der Wärmemengenzufuhr zu Gasverbrauchseinrichtungen anhand von in der Zeichnung dargestellten Ausführungsbeispielen näher erläutert.

In der Zeichnung zeigen:
- Fig. 1: eine schematische Ansicht einer Anordnung zur Durchführung eines ersten Ausführungsbeispiels der erfindungsgemäßen Verwendung des Verfahrens zur Brennwertmessung;
- Fig. 2: eine schematische Ansicht einer Anordnung zur Durchführung eines zweiten Ausführungsbeispiels der erfindungsgemäßen Verwendung;
- Fig. 3: eine schematische Ansicht einer Anordnung zur Durchführung eines dritten Ausführungsbeispiels der erfindungsgemäßen Verwendung;
- Fig. 4: eine schematische Ansicht einer Anordnung zur Durchführung eines vierten Ausführungsbeispiels der erfindungsgemäßen Verwendung;
- Fig. 5: eine schematische Ansicht einer Anordnung zur Durchführung eines fünften Ausführungsbeispiels der erfindungsgemäßen Verwendung;
- Fig. 6: eine schematische Ansicht einer Anordnung zur Durchführung eines sechsten Ausführungsbeispiels der erfindungsgemäßen Verwendung.

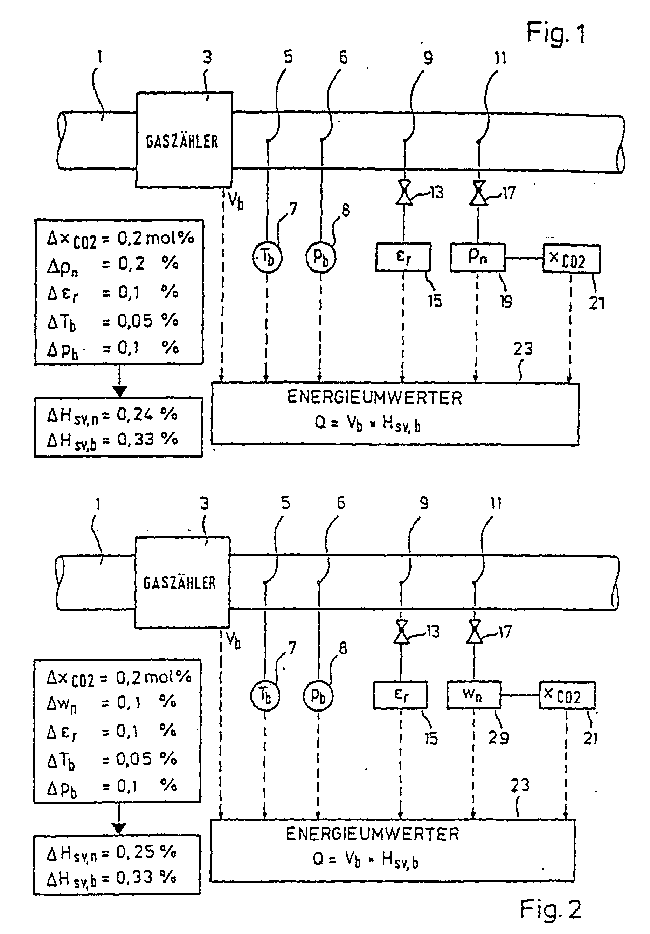

Fig. 1 zeigt eine Brenngasleitung 1, in der ein Gaszähler 3 angeordnet ist. In der Brenngasleitung 1 sind außerdem zwei Meßstellen 5 und 6 angeordnet. Der Meßstelle 5 ist ein Temperatursensor 7 und der Meßstelle 6 ein Drucksensor 8 zugeordnet. Ferner sind in der Brenngasleitung zwei Entnahmestellen 9 und 11 vorgesehen. Die Entnahmestelle 9 ist über ein Druckreduzierventil 13 mit einer Meßeinrichtung zur Messung der Dielektrizitätskonstanten 15 verbunden. Die Entnahmestelle 11 ist über ein Druckreduzierventil 17 mit einer Meßeinrichtung zur Messung der Dichte 19 verbunden. Die Meßeinrichtung 19 ist über eine Brenngasleitung mit einer Meßeinrichtung 21 zur Bestimmung des Kohlenstoffdioxidanteils verbunden.

Die Signalausgänge des Gaszählers 3, des Temperatursensors 7, des Drucksensors 8 sowie der Meßeinrichtungen 15, 19 und 21 sind mit den Eingängen eines Energieumwerters 23 verbunden.

Im Betriebszustand mißt der Gaszähler 3 den Volumenstrom und die Zeit und berechnet daraus das Volumen des zugeführten Brenngases. Der Temperatursensor 7 mißt die Temperatur und der Drucksensor 8 den Druck in der Brenngasleitung 1. Die Meßeinrichtung 15 mißt die Dielektrizitätskonstante unter Referenzbedingungen, und zwar z.B. bei einem Druck von einem MPa und einer Temperatur von 288,15 K. Die Dichtemessung wird in der Meßeinrichtung 19 unter Normbedingungen durchgeführt. Nach der Dichtemessung wird an dem an der Entnahmestelle 11 entnommenen Brenngasteilstrom der Kohlenstoffdioxidanteil gemessen. Alle ermittelten Meßwerte werden an den Energieumwerter 23 gesendet. Dieser berechnet zunächst korrelativ den Brennwert bei Betriebsbedingungen H_{SV,b}. Anschließend berechnet der Energieumwerter 23 durch Multiplikation des Brennwertes H_{SV,b} mit dem Volumen bei Betriebsbedingungen V_{b} die Wärmemenge Q.

Fig. 2 zeigt eine Anordnung zur Durchführung eines zweiten Ausführungsbeispiels der Erfindung. Die Anordnung gemäß Fig. 2 unterscheidet sich lediglich dadurch von der Anordnung gemäß Fig. 1, daß statt der Meßeinrichtung 19 zur Dichtmessung eine Meßeinrichtung 29 zur Messung der Schallgeschwindigkeit unter Normbedingungen vorgesehen ist. Die Wärmemenge wird von dem Energieumwerter 23 bei diesem Ausführungsbeispiel auf der Basis des Volumes, der Temperatur und des Druckes bei Betriebsbedingungen, der Dielektrizitätskonstanten unter Referenzbedingungen, der Schallgeschwindigkeit unter Normbedingungen sowie des Kohlenstoffdioxidanteils berechnet.

Da in den beiden Ausführungsbeispielen gemäß Fig. 1 und Fig. 2 die Meßeinrichtungen für die Dielektrizitätskonstante, die Schallgeschwindigkeit und den Druck außerhalb der Brenngasleitung angeordnet sind, ist die Wartung dieser Einrichtungen sowie eine eventuelle Reparatur ohne besonderen technischen Aufwand durchzuführen. Außerdem sind nur verhältnismäßig wenige Referenzzyklen erforderlich, da die Dielektrizitätskonstante und die Schallgeschwindigkeit unter vorgegebenen Bedingungen gemessen werden. Es besteht somit eine direkte Korrelation zwischen einer Änderung der Dielektrizitätskonstanten und der Schallgeschwindigkeit oder der Dichte und einer Änderung der Gaszusammensetzung. Das Ausführungsbeispiel gemäß Fig. 2 ist besonders kostengünstig zu realisieren, da die Schallgeschwindigkeit einfacher und günstiger zu messen ist als die Dichte des Brenngases.

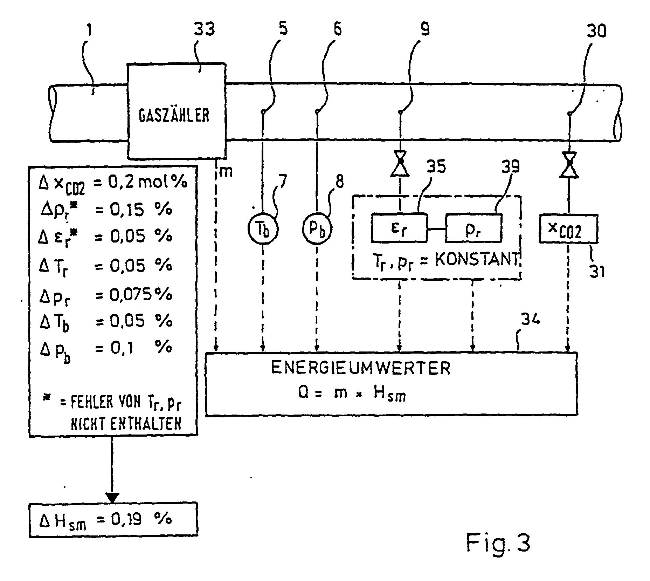

In Fig. 3 ist ein drittes Ausführungsbeispiel der Erfindung schematisch dargestellt. Die Anordnung unterscheidet sich von der Anordnung gemäß Fig. 1 dadurch, daß statt des Volumenzählers ein Massenzähler 33 zur Messung der Masse des zugeführten Brenngases vorgesehen ist. Ferner ist eine separate Entnahmestelle 30 der Meßeinrichtung zur Bestimmung des Kohlenstoffdioxidanteils 31 zugeordnet ist. Außerdem sind die Meßeinrichtungen zur Dielektrizitätsmessung 35 und zur Dichtmessung 39 in einer gemeinsamen Meßumgebung angeordnet, in der eine vorgegebene Temperatur und ein vorgegebener Druck herrscht.

Der Energieumwerter 34 berechnet zunächst korrelativ den Brennwert H_{Sm}. Anschließend berechnet der Energieumwerter 34 durch Multiplikation des Brennwertes H_{Sm} mit der Masse m die Wärmemenge Q.

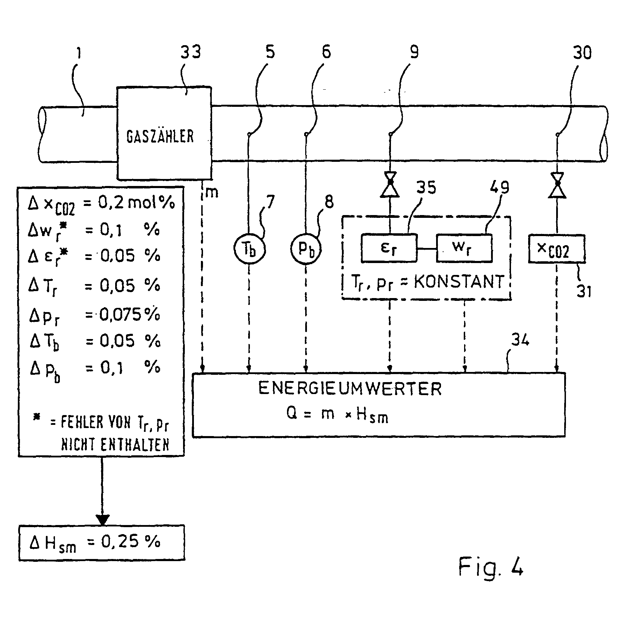

Das in Fig. 4 dargestellte vierte Ausführungsbeispiel der Erfindung unterscheidet sich von dem in Fig. 3 dargestellten dritten Ausführungsbeispiel lediglich dadurch, daß statt einer Meßeinrichtung zur Dichtmessung 39 eine Meßeinrichtung zur Messung der Schallgeschwindigkeit 49 in der gemeinsamen Meßumgebung vorgesehen ist.

Das dritte und das vierte Ausführungsbeispiel haben wie die beiden ersten Ausführungsbeispiele den Vorteil, daß wenige Referenzzyklen durchgeführt werden müssen, um die Wärmemenge zuverlässig zu bestimmen. Ferner hat die gemeinsame Meßumgebung für die Dielektrizitätsmessung und die Dichte- bzw. Schallgeschwindigkeitsmessung den Vorteil, daß nur eine Druck- und Temperaturmessung zusätzlich zur Druck- und Temperaturmessung in der Brenngasleitung benötigt wird. Im Vergleich zu den beiden ersten Ausführungsbeispielen kann außerdem eine Thermostat eingespart werden. Das dritte Ausführungsbeispiel weist eine besonders hohe Genauigkeit bei der Bestimmung der Wärmemenge auf und ist daher besonders vorteilhaft.

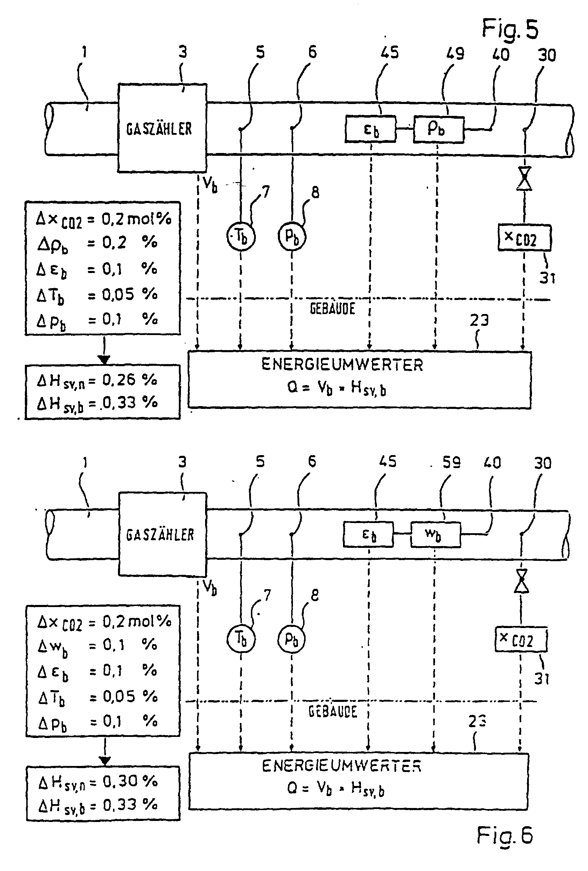

In Fig. 5 ist ein fünftes Ausführungsbeispiel der Erfindung veranschaulicht. Die Anordnung gemäß Fig. 5 unterscheidet sich von der Anordnung in Fig. 3 dadurch, daß in der Brenngasleitung eine Meßstelle 40 vorgesehen ist, der eine Meßeinrichtung 45 zur Dielektrizitätsmessung bei Betriebsbedingungen und eine Meßeinrichtung 49 zur Dichtmessung bei Betriebsbedingungen zugeordnet.sind. Alle Meßgrößen, bis auf den Kohlenstoffdioxidanteil, werden bei diesem Ausführungsbeispiel unter Betriebsbedingungen gemessen. Außerdem wird statt der bei den Ausführungsbeispielen gemäß Fig. 3 und 4 gemessenen Masse das Volumen gemessen. Die Meßgrößen Volumen, Temperatur, Druck, Dielektrizitätskonstante sowie Dichte unter Betriebsbedingungen werden an den Energieumwerter 23 gesendet.

In Fig. 6 ist ein sechstes Ausführungsbeispiel der Erfindung dargestellt. Die Anordnung gemäß Fig. 6 unterscheidet sich von der Anordnung gemäß Fig. 5 lediglich dadurch, daß in der Brenngasleitung 1 anstelle der Meßeinrichtung 49 zur Dichtemessung eine Meßeinrichtung 59 zur Schallgeschwindigkeitsmessung vorgesehen ist.

Das fünfte und sechste Ausführungsbeispiel haben den Vorteil, daß aufgrund der Messung der Dielektrizitätskonstanten sowie der Dichte bzw. der Schallgeschwindigkeit unter Betriebsbedingungen keine Referenzbedingungen einzustellen sind. Somit vereinfacht sich die Anordnung insofern, als auf Thermostate vollständig verzichtet werden kann.

Außerdem entfallen die zur Einstellung der Referenzbedingungen benötigten Temperatur- und Drucksensoren vollständig. Eine Verringerung des Druckes ist nur noch zur Bestimmung des Kohlenstoffdioxidanteils in der Meßeinrichtung 31 erforderlich. Schließlich haben die Ausführungsbeispiele gemäß Fig. 5 und Fig. 6 den Vorteil, daß das Verfahren im Freien durchgeführt werden kann, da alle Meßeinrichtungen der Brenngasleitung unmittelbar zugeordnet sind. In den dargestellten Ausführungsbeispielen sind die Meßeinrichtungen über Signalleitungen mit dem in einem Gebäude angeordneten Energieumwerter 23 verbunden. Der Platzbedarf zur Durchführung des erfindungsgemäßen Verfahrens ist bei diesen beiden Ausführungsbeispielen sehr gering.

Im Rahmen des Erfindungsgedankens sind zahlreiche Weiterbildungen möglich. Insbesondere kann die zeitliche Reihenfolge der Messungen zur Bestimmung der verschiedenen Meßgrößen beliebig variiert werden. Auch die Referenzbedingungen können frei gewählt werden. Schließlich können nicht nur die Dielektrizitätskonstante und die Dichte bzw. Schallgeschwindigkeit, sondern auch der Kohlenstoffdioxidanteil in der gemeinsamen Meßumgebung bestimmt werden.

## Patentansprüche

1. Verfahren zur verbrennungslosen Messung des Brennwertes von Brenngas, das durch eine Brenngasleitung strömt, wobei
a) die Dielektrizitätskonstante des Brenngases unter Referenz- oder Betriebsbedingungen und die Dichte oder die Schallgeschwindigkeit des Brenngases unter Referenz- oder Betriebsbedingungen erfasst werden und
b) aus diesen zwei Parametern der Brennwert abgeleitet wird,
c) den Verfahrensschritten a) und b) mehrere Messzyklen vorgeschaltet werden, bei denen der Schritt a) mit mehreren Referenzgasen bekannten Brennwerts durchgeführt wird; aus dem Verhältnis der bei den Referenzgasen erfassten verschiedenen Messsignale eine der Zahl der Messzyklen entsprechende Zahl von Referenzsignalmustem in Zuordnung zu den bekannten Brennwerten gespeichert wird; und das Signalmuster aus einem späteren Messzyklus an Brenngas unbekannten Brennwertes mit den Referenzsignalmustern zur Zuordnung eines bestimmten Brennwertes verglichen wird.

2. Verfahren nach Anspruch 1, **dadurch gekennzeichnet, dass** die Dielektrizitätskonstante und die Dichte oder die Schallgeschwindigkeit unter Referenzbedingungen in einer gemeinsamen Messumgebung erfasst werden.

3. Verfahren nach Anspruch 1 oder 2, **dadurch gekennzeichnet, dass** als Referenzbedingungen für die Messung der Dichte oder der Schallgeschwindigkeit und/oder der Dielektrizitätskonstanten Normbedingungen eingestellt werden.

4. Verfahren nach einem der Ansprüche 1 bis 3, **dadurch gekennzeichnet, dass** als Referenzbedingung für die Messung der Dielektrizitätskonstanten ein Referenzdruck von wenigstens 1 MPa eingestellt wird.

5. Verfahren nach einem der Ansprüche 1 bis 4, **dadurch gekennzeichnet, dass** im Schritt a) zusätzlich wenigstens eine der Messgrößen Temperatur, Druck oder der Anteil mindestens eines Inertgases erfasst wird.

6. Verfahren nach Anspruch 5, **dadurch gekennzeichnet, daß** ein Teilstrom des Brenngases zur Messung der Dichte oder der Schallgeschwindigkeit unter Referenzbedingungen abgezweigt wird und dass an diesem Teilstrom die Erfassung des Anteils mindestens eines Inertgases durchgeführt wird.

7. Verfahren nach Anspruch 5 oder 6, **dadurch gekennzeichnet, dass** der Kohlenstoffdioxidanteil erfasst wird.

8. Verwendung des Verfahrens nach einem der Ansprüche 1 bis 7 zur verbrennungslosen Messung und/oder Regelung der Wärmemengenzufuhr zu Gasverbrauchseinrichtungen, wobei im Schritt a) zusätzlich das Brenngas bzw. ein Teilstrom des Brenngases durch einen Volumen- oder Massenzähler geleitet und das Volumen bzw. die Masse des zugeführten Brenngases gemessen wird.

## Claims

1. Method for the combustion-free measurement of the gross calorific value of fuel gas flowing through a fuel gas line, wherein
a) the dielectric constant of the fuel gas is recorded under reference or operating conditions and the density or the velocity of sound of the fuel gas is recorded under reference or operating conditions, and
b) the gross value is determined from these two parameters,
c) process steps a) and b) are preceded by several measurement cycles during which step a) is carried out with several reference gases of known gross calorific values; on the basis of the ratio of the different measuring signals recorded from the reference gases, a number of reference signal patterns corresponding to the number of the measurement cycles is allocated to the known gross calorific values and stored; the signal pattern of a later measurement cycle on a fuel gas of an unknown gross calorific value is compared with the reference signal patterns so a specific gross calorific value can be allocated.

2. Method according to claim 1, **characterised in that** the dielectric constant and the density or the velocity of sound under reference conditions are recorded in a common measuring environment.

3. Method according to claims 1 or 2, **characterised in that** normal conditions are set as reference conditions for the measurement of the density or the velocity of sound and/or the dielectric constants.

4. Method according to any one of claims 1 to 3, **characterised in that** a reference pressure of at least 1 MPa is set as the reference condition for the measurement of the dielectric constants.

5. Method according to any one of claims 1 to 4, **characterised in that** in step a) at least one of the measurands temperature, pressure or the percentage of at least one inert gas, preferably carbon dioxide, is additionally recorded.

6. Method according to claim 5, **characterised in that** part of the fuel gas flow is removed for measuring the density or the velocity of sound under reference conditions, and that the percentage of at least one inert gas is measured on this part of the fuel gas flow.

7. Method according to one of claims 5 or 6, **characterised in that** the percentage of carbon dioxide is recorded.

8. Use of the method according to any one of claims 1 to 7 for the combustion-free measurement and/or control of the quantity of heat supplied to gas consumption appliances, in particular natural gas appliances, the fuel gas or part of the fuel gas flow being also fed through a volumetric or mass flow meter in step a) and the volume or mass of the fuel gas supplied being measured.

## Revendications

1. Procédé pour mesurer sans combustion le pouvoir calorifique supérieur d'un gaz combustible s'écoulant dans une conduite de gaz combustible, où
a) la constante diélectrique du gaz combustible est déterminée dans des conditions de référence ou d'exploitation et la masse volumique ou la vitesse du son du gaz combustible est déterminée dans des conditions de référence ou d'exploitation, et
b) le pouvoir calorifique supérieur est déduit de ces deux paramètres,
c) plusieurs cycles de mesure précèdent les pas a) et b) du procédé, où le pas a) est réalisé avec plusieurs gaz de référence dont les pouvoirs calorifiques supérieurs sont connus ; à partir du rapport des différents signaux de mesure saisis avec les gaz de référence, un chiffre pour des modèles de signaux de référence, correspondant au chiffre des cycles de mesure, est mis en mémoire par affectation aux pouvoirs calorifiques supérieurs connus ; et le modèle de signaux issu d'un cycle de mesure ultérieur avec un gaz combustible dont le pouvoir calorifique supérieur n'est pas connu est comparé avec les modèles de signaux de référence pour l'affectation d'un pouvoir calorifique supérieur déterminé.

2. Procédé suivant la revendication 1, **caractérisé par le fait que** la constante diélectrique et la masse volumique ou la vitesse du son sont saisis dans des conditions de référence dans un environnement de mesurage commun.

3. Procédé suivant l'une des revendications 1 ou 2, **caractérisé par le fait que** des conditions normalisées sont ajustées comme conditions de référence pour le mesurage de la masse volumique ou de la vitesse du son et/ou de la constante diélectrique.

4. Procédé suivant l'une des revendications 1 à 3, **caractérisé par le fait qu'**une pression de référence d'au moins 1 MPa est ajustée comme condition de référence pour le mesurage de la constante diélectrique.

5. Procédé suivant l'une des revendications 1 à 4, **caractérisé par le fait que** pour le pas a) au moins une des mesurandes de température, de pression ou la teneur d'au moins un gaz inerte est saisie.

6. Procédé suivant la revendication 5, **caractérisé par le fait qu'**un flux partiel du gaz combustible est dérivé pour le mesurage de la masse volumique ou de la vitesse du son dans des conditions de référence et que dans ce flux partiel est saisie la teneur d'au moins un gaz inerte.

7. Procédé suivant l'une des revendications 5 ou 6, **caractérisé par le fait que** la teneur en dioxyde de carbone est saisie.

8. Application du procédé suivant l'une des revendications 1 à 7 pour mesurer et/ou régler sans combustion la quantité d'énergie apportée à des appareils d'utilisation du gaz, où pour le pas a) le gaz combustible resp. un flux partiel du gaz combustible passe de plus par un compteur volumétrique ou massique et que le volume resp. la masse du gaz combustible apporté est mesuré.
